(19) Europäisches Patentamt
European Patent Office
Office européen des brevets

(11) Veröffentlichungsnummer: **0 240 818 B1**

(12) **EUROPÄISCHE PATENTSCHRIFT**

(45) Veröffentlichungstag der Patentschrift: **04.12.91**

(51) Int. Cl.$^5$: **C07C 211/15**

(21) Anmeldenummer: **87104213.1**

(22) Anmeldetag: **21.03.87**

Die Akte enthält technische Angaben, die nach dem Eingang der Anmeldung eingereicht wurden und die nicht in dieser Patentschrift enthalten sind.

# (54) Fluorierte tertiäre Butylamine.

(30) Priorität: **04.04.86 DE 3611195**

(43) Veröffentlichungstag der Anmeldung:
**14.10.87 Patentblatt 87/42**

(45) Bekanntmachung des Hinweises auf die Patenterteilung:
**04.12.91 Patentblatt 91/49**

(84) Benannte Vertragsstaaten:
**BE CH DE FR GB IT LI NL**

(56) Entgegenhaltungen:
**EP-A- 0 132 729**
**EP-A- 0 132 733**
**EP-A- 0 132 734**

(73) Patentinhaber: **BAYER AG**
**W-5090 Leverkusen 1 Bayerwerk(DE)**

(72) Erfinder: **Baasner, Bernd, Dr.**
**Hamberger Strasse 27 d**
**W-5090 Leverkusen 3(DE)**
Erfinder: **Hagemann, Hermann, Dr.**
**Kandinsky-Strasse 52**
**W-5090 Leverkusen(DE)**
Erfinder: **Schwamborn, Michael, Dr.**
**Von-Lohe-Strasse 9**
**W-5000 Köln 80(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Anmeldung betrifft neue fluorierte tertiäre Butylamine und mehrere Verfahren zu ihrer Herstellung.

Es wurden neue fluorierte tertiäre Butylamine der Formel (I)

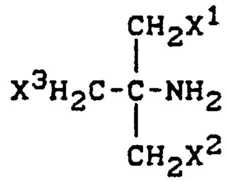

(I)

gefunden,
in welcher die Reste $X^1$ bis $X^3$ gleich oder verschieden sein können und für Wasserstoff oder Fluor stehen, wobei wenigstens einer der Reste $X^1$ bis $X^3$ für Fluor stehen muß.

Man erhält die fluorierten tertiären Butylamine der Formel (I)

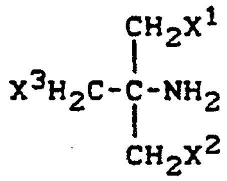

(I)

in welcher die Reste $X^1$ bis $X^3$ gleich oder verschieden sein können und für Wasserstoff oder Fluor stehen, wobei wenigstens einer der Reste $X^1$ bis $X^3$ für Fluor steht,
wenn man in der Verfahrensvariante A)

(a) die halogenierten Pivalsäurehalogenide der allgemeinen Formel (II)

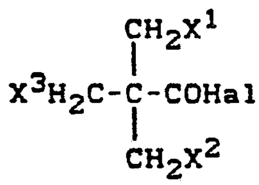

(II),

in welcher $X^1$ bis $X^3$ die oben angegebene Bedeutung haben und Hal für Fluor oder Chlor stehen,
mit einem Azidgruppen übertragenden Reagenz in die fluorierten Pivalsäureazide der allgemeinen Formel (III)

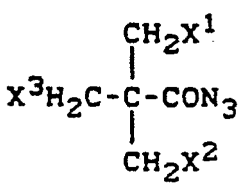

(III),

in welcher $X^1$ bis $X^3$ die oben angegebene Bedeutung haben, umsetzt und

(b) die gegebenenfalls isolierten Verbindungen der allgemeinen Formel (III) thermisch zu den fluorierten Isocyanaten der allgemeinen Formel (IV)

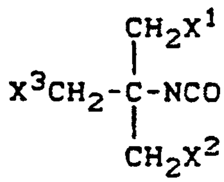

(IV),

in welcher $X^1$ bis $X^3$ die oben angegebene Bedeutung haben, zersetzt und

(c) die gegebenenfalls isolierten Verbindungen der allgemeinen Formel (IV) durch saure Hydrolyse in die fluorierten tertiären Butylamin-Säureadditionssalze der allgemeinen Formel (V)

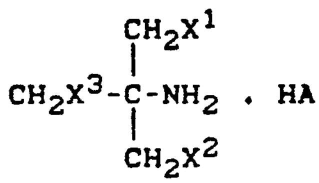

(V),

in welcher $X^1$ bis $X^3$ die oben angegebene Bedeutung haben, und A für den einwertigen Rest einer Säure steht, umwandelt und

(d) die gegebenenfalls isolierten Verbindungen der allgemeinen Formel (V) durch Behandlung mit Base in die fluorierten tertiären Butylamine der allgemeinen Formel (I) überführt,

oder

in der Verfahrensvariante B)

die Verbindungen der Formel (IV)

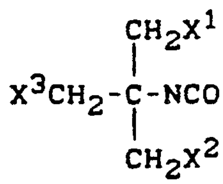

(IV),

durch alkalische Hydrolyse direkt in die fluorierten tertiären Butylamine der allgemeinen Formel (I) überführt,

oder in der Verfahrensvariante C)

Pivalsäureamide der allgemeinen Formel (VI)

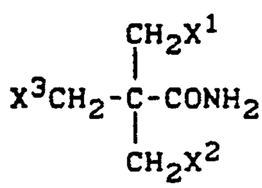

(VI),

in welcher $X^1$ bis $X^3$ die oben angegebene Bedeutung haben,

durch Umsetzung mit Hypohalogenit in die fluorierten tertiären Butylamine der allgemeinen Formel (I) überführt.

Die neuen Verbindungen der allgemeinen Formel (I) eignen sich als wertvolle Zwischenprodukte für Synthesen in der organischen Chemie, z.B. auf den Gebieten der Farbstoffe, Pharmazeutika, Pflanzenschutzmittel, Kunststoffe und Kautschukhilfsmittel.

Insbesondere geeignet sind die neuen Verbindungen der Formel (I) für die Herstellung von neuen Benzoesäureamiden, die insektizide Wirkung aufweisen.

Folgende Synthese mag hier beispielhaft aufgeführt werden:

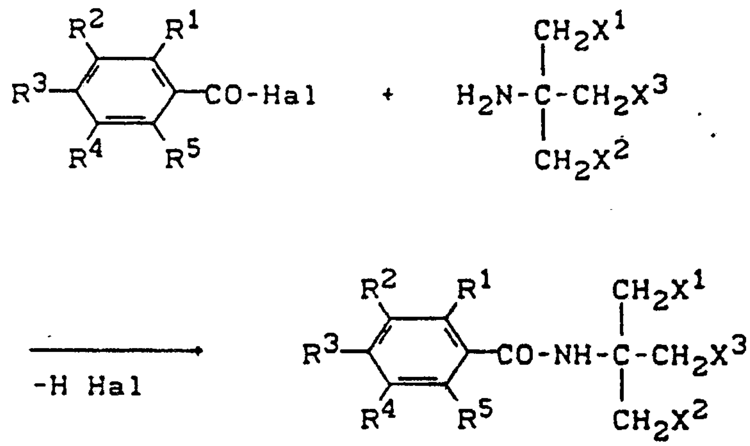


wobei $X^1$ bis $X^3$ die oben angegebene Bedeutung haben und die Reste $R^1$, $R^2$, $R^3$, $R^4$ und $R^5$ gleich oder verschieden sind und für Wasserstoff, Halogen, Nitro, Alkyl, Halogenalkyl, Halogenalkoxy oder Halogenalkylthio stehen.

Folgende Verbindungen der allgemeinen Formel (I) kommen in Frage:
2-Fluor-1,1-dimethyl-ethylamin,
2-Fluor-1-(monofluormethyl)-1-methyl-ethylamin,
2-Fluor-1,1-bis-(monofluormethyl)-ethylamin.

Verwendet man in der Verfahrensvariante A) Monofluorpivaloylchlorid als Ausgangsprodukt so kann die Umsetzungssequenz (a) bis (d) wie folgt dargestellt werden:

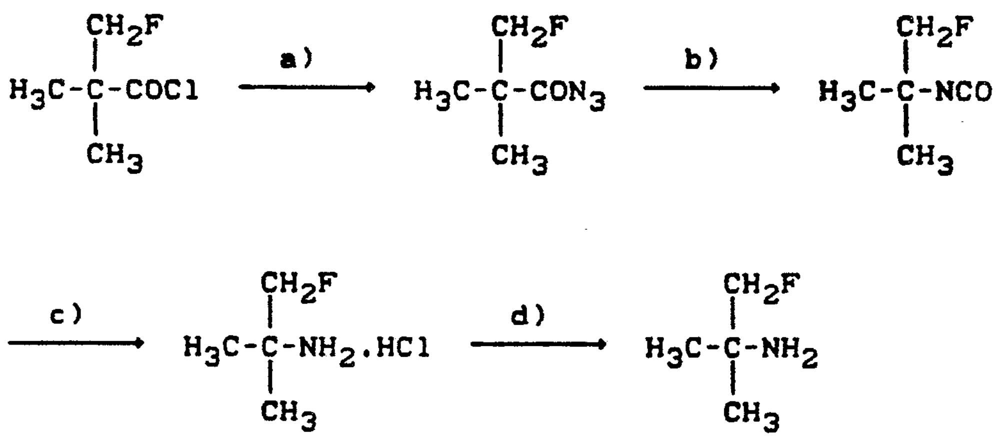


Verwendet man in der Verfahrensvariante B) 2-Fluor-1,1-dimethylethylisocyanat als Ausgangsverbindung und hydrolysiert mit wäßriger Natronlauge, so kann die Umsetzung wie folgt dargestellt werden:

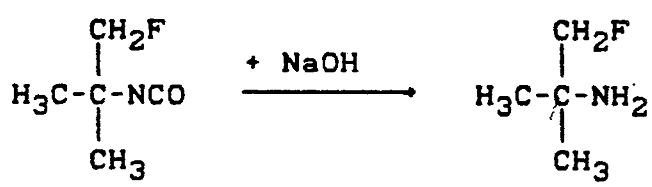


Verwendet man in der Verfahrensvariante C) Monofluorpivaloylamid als Ausgangsprodukt und setzt im Sinne eines Hofmann-Abbaus mit Hypohalogenit um, so kann die Umsetzung wie folgt dargestellt werden:

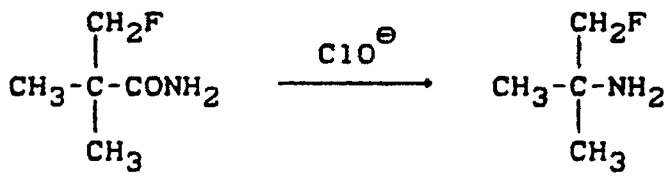

Für die Herstellungsvariante A) gilt:

Die in den einzelnen Verfahrensstufen (a) bis (d) erhaltenen Verbindungen der allgemeinen Formeln (I) bis (V) können entweder nach üblichen Methoden isoliert oder ohne Isolierung direkt in der nächsten Stufe weiterverarbeitet werden.

Die als Ausgangsstoffe einzusetzenden fluorierten Pivalsäurehalogenide der allgemeinen Formel (I) sind aus der DE-OS 33 26 875 bekannt. In der DE-OS 33 26 873 wird beschrieben, daß das 2-Fluor-1-(monofluormethyl)-1-methyl-propionsäurefluorid in das entsprechende Azid überführt, dieses ohne Zwischenisolierung in das 2-Fluor-1-(mono-fluormethyl)-1-methylisocyanat umgewandelt und dieses schließlich ohne Zwischenisolierung mit 2-Propinalkohol zum N-(1,1-Bisfluormethyl-ethyl)-carbamidsäure-0-2-propinylester, der als Schädlingsbekämpfungsmittel verwendbar ist, umgesetzt werden kann.

Die Reaktionsstufen (a) bis (d) können nach den üblichen Methoden der organischen Chemie durchgeführt werden.

In der Reaktionsstufe (a) werden die fluorierten Pivalsäurehalogenide der allgemeinen Formel (II) mit einem Azidgruppen übertragenden Reagenz in die Azide der allgemeinen Formel (III) überführt (s. z.B. Houben-Weyl, Methoden der organischen Chemie, Band IX/1, 862 - 872 (1957)). Hierzu werden die fluorierten Pivalsäurehalogenide z.B. in Aceton gelöst und mit einer etwa molaren Menge an Natriumazid (in wäßriger Lösung) bei etwa 0 °C bis etwa +20 °C umgesetzt. Anschließend wird mit einem inerten organischen Lösungsmittel, wie etwa Toluol, extrahiert, die organische Phase abgetrennt und getrocknet. Aus dieser Lösung können die Azide destillativ abgetrennt werden.

Ein vorteilhaftes Verfahren besteht darin, die fluorierten Pivalsäurehalogenide der allgemeinen Formel (II) nach dem Verfahren, wie es in Synthesis 1972, 551 - 553, beschrieben ist, mit Trimethylsilylazid in das Azid der allgemeinen Formel (III) zu überführen. Auch danach kann das Azid destillativ isoliert werden.

Die so gewonnenen Azide der allgemeinen Formel (III) können zur weiteren Umsetzung zum Isocyanat der allgemeinen Formel (IV) in reiner Form, vorzugsweise aber in Form ihrer verdünnten Lösungen, wie sie im vorangegangenen Schritt angefallen sind, eingesetzt werden. Die Umwandlung der Azide in die Isocyanate erfolgt ebenfalls nach bekannten Verfahren (s. z.B. Houben-Weyl, Methoden der organischen Chemie, Band XI/1, 862 - 872 (1957)), indem man das Azid in einem inerten organischen Lösungsmittel wie beispielsweise Benzol oder Toluol bis zum Ende der Stickstoffentwicklung erhitzt. Anschließend kann das Isocyanat destillativ in reiner Form erhalten werden.

Das so gewonnene Isocyanat der allgemeinen Formel (IV) wird in der nachfolgenden Reaktionsstufe in das Amin-Säureadditionssalz der allgemeinen Formel (V) überführt. Hierbei wird das Isocyanat der allgemeinen Formel (IV) vorzugsweise in wäßriger Salzsäure beliebiger Konzentration hydrolysiert.

Diese Hydrolyse kann in der wäßrigen Salzsäure oder aber in Gegenwart eines inerten organischen Lösungsmittels bei Temperaturen zwischen 0° und +100 °C durchgeführt werden. Die Reaktionszeit beträgt zwischen 0,5 und 18 Stunden. Nach dem Abdestillieren der flüchtigen Bestandteile bei Normaldruck oder vermindertem Druck bleibt als Rückstand das reine Amin-Hydrochlorid der allgemeinen Formel (V). Daraus kann das freie Amin der allgemeinen Formel (I) durch Behandlung mit einer Lauge zur Neutralisation der Salzsäure aus dem Hydrochlorid gewonnen werden. Das Amin wird durch Abdestillieren aus diesem Rohgemisch in reiner Form gewonnen. Anstelle der Salzsäure kann zur Hydrolyse auch jede andere geeignete Mineralsäure (wie z.B. Schwefelsäure) verwendet werden. Es ist aber auch möglich, das Amin der allgemeinen Formel (I) aus diesem Rohgemisch mit einem inerten organischen Lösungsmittel zu extraktieren und das Amin aus diesem Extrakt durch Destillation zu isolieren. Es ist weiterhin möglich, das freie Amin der allgemeinen Formel (I) ohne Zwischenisolierung des Amin-Hydrochlorids der allgemeinen Formel (V) aus dem neutralisierten oder alkalisch gestellten Rohgemisch der Isocyanat-Hydrolyse destillativ oder extraktiv zu isolieren. Darüber hinaus ist es möglich, aus den Isocyanaten der allgemeinen Formel (IV) durch basische Verseifung mit Alkalilaugen das Amin (I) in Freiheit zu setzen und aus diesem Rohgemisch nach den bereits erläuterten Methoden durch Destillation oder Extraktion mit anschließender Destillation in reiner Form zu isolieren.

Weiterhin ist es möglich, die Pivalsäureamide der allgemeinen Formel (VI)

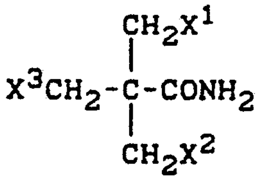

(VI),

in welcher $X^1$ bis $X^3$ die oben angegebene Bedeutung haben, durch Hofmann-Abbau mit Hypohalogenit gemäß bekannten Verfahren (s. z.B. Houben-Weyl, Methoden der organischen Chemie, Bd. XI/1, S. 854 - 862 (1957)) in die tertiären Butylamine der allgemeinen Formel (I) zu überführen.

Die Verbindungen der allgemeinen Formel (VI) sind ebenfalls aus der DE-OS 33 26 874 bekannt. Sie werden durch Umsetzung der Carbonsäurehalogenide der allgemeinen Formel (II) mit Ammoniak hergestellt.

## Beispiele

### Beispiel 1:

2-Fluor-1,1-dimethyl-ethylisocyanat aus Monofluorpivaloylchlorid mit Trimethylsilylazid

69,5 g (0,5 Mol) Monofluorpivaloylchlorid werden in etwa 10 Minuten zu einer Lösung von 70 g Trimethylsilylazid in 300 ml Toluol getropft. Es wird 30 Min. gerührt, wobei die Temperatur bis auf 45 °C ansteigt und Gasentwicklung beginnt. Anschließend wird die Temperatur langsam gesteigert (in ca. 5 Stunden bis auf 90 °C) und gerührt, bis die Gasentwicklung beendet ist. Der Fortgang der Reaktion wird IR-spektroskopisch verfolgt (Abnahme der Azid-Bande bei 2130 $cm^{-1}$). Aus dieser Reaktionslösung wird das Isocyanat abdestilliert. Man erhält 52,1 g (89 % der Theorie) 2-Fluor-1,1-dimethyl-ethylisocyanat vom Siedepunkt 98 - 100 °C (bei Normaldruck).

### Beispiel 2:

2-Fluor-1,1-dimethyl-ethylisocyanat aus Monofluorpivaloylfluorid mit Natriumazid

Zu 12,2 g (0,1 Mol) Monofluorpivaloylfluorid in 200 ml Aceton wird unter Eisbadkühlung bei 0 °C bis +5 °C eine Lösung von 6,6 g Natriumazid in 20 ml Wasser zugetropft.

Es wird 1 Stunde nachgerührt, mit 300 ml Eiswasser versetzt und dreimal mit je 100 ml Toluol extrahiert. Die organische Phase wird einmal mit 200 ml Wasser gewaschen und mit Magnesiumsulfat getrocknet. Die so dargestellte Azid-Lösung wird anschließend langsam aufgeheizt (in ca. 3 Stunden auf 90 °C) und nachgerührt, bis die Gasentwicklung beendet ist (IR-spektroskopische Verfolgung der Reaktion wie in Beispiel 1 beschrieben). Aus dieser Lösung kann das Isocyanat abdestilliert werden. Man erhält 9,5 g (81 % der Theorie) 2-Fluor-1,1-dimethyl-ethylisocyanat vom Siedepunkt 99 - 100 °C (bei Normaldruck).

### Beispiel 3:

2-Fluor-1,1-dimethyl-ethylisocyanat aus Monofluorpivaloylfluorid mit Trimethylsilylazid

Analog zu Beispiel 1 werden 61 g (0,5 Mol) Monofluorpivaloylfluorid mit Trimethylsilylazid umgesetzt. Nach Beendigung der Gasentwicklung wird aus der Lösung destilliert. Man erhält 53,8 g (92 % der Theorie) 2-Fluor-1,1-dimethyl-ethylisocyanat vom Siedepunkt 96 - 99 °C (bei Normaldruck).

### Beispiel 4:

2-Fluor-1,1-dimethyl-ethylisocyanat aus Monofluorpivaloylchlorid mit Natriumazid

Analog zu Beispiel 2 werden 13,9 g (0,1 Mol) Monofluorpivaloylchlorid mit Natriumazid umgesetzt. Man erhält 9 g (77 % der Theorie) 2-Fluor-1,1-dimethyl-ethylisocyanat vom Siedepunkt 100 - 101 °C (bei Normaldruck).

### Beispiel 5:

2-Fluor-1-(monofluormethyl)-1-methyl-ethylisocyanat aus 3-Fluor-2-(monofluormethyl)-2-methylpropansäurefluorid

Analog zu Beispiel 3 werden 70 g (0,5 Mol) 3-Fluor-2-(monofluormethyl)-2-methylpropansäurefluorid mit 70 g Trimethylsilylazid umgesetzt, jedoch in Benzol als Lösungsmittel. Die Reaktionsmischung wird bis zum Ende der Gasentwicklung zum Rückfluß erhitzt. Anschließend werden über eine kurze Füllkörperkolonne die flüchtigen Bestandteile, dann das Isocyanat abdestilliert. Man erhält 56 g (83 %) 2-Fluor-1-(monofluormethyl)-1-methyl-ethylisocyanat vom Siedepunkt 122 - 124° C (bei Normaldruck).

Beispiel 6:

2-Fluor-1,1-bis-(monofluormethyl)-ethylisocyanat aus 3-Fluor-2,2-bis-(monofluormethyl)-propansäurechlorid mit Trimethylsilylazid

Analog zu Beispiel 1, jedoch in Benzol als Lösungsmittel, werden 87,25 g (0,5 Mol) 3-Fluor-2,2-bis-(monofluormethyl)-propansäurechlorid mit 70 g Trimethylsilylazid umgesetzt. Die Reaktionsmischung wird bis zum Ende der Gasentwicklung zum Rückfluß erhitzt. Anschließend werden über eine kurze Füllkörperkolonne die flüchtigen Bestandteile, dann das Isocyanat abdestilliert. Man erhält 65,4 g (85,5 % der Theorie) 2-Fluor-1,1-bis-(monofluormethyl)-ethylisocyanat vom Siedepunkt 143 - 146° C (bei Normaldruck).

Beispiel 7:

2-Fluor-1,1-(dimethyl)-ethylamin-Hydrochlorid

Zu 400 ml konz. Salzsäure werden in ca. 30 Minuten 58,5 g (0,5 Mol) 2-Fluor-1,1-dimethyl-ethylisocyanat, wie es nach den Beispielen 1 bis 4 gewonnen werden kann, in 300 ml Toluol zugetropft (Gasentwicklung). Anschließend wird bei 60° C bis zum Ende der Gasentwicklung gerührt (ca. eine Stunde). Nach Abdestillieren der flüchtigen Bestandteile erhält man das Produkt 2-Fluor-1,1-(dimethyl)-ethylamin-Hydrohlorid. Ausbeute 60,6 g (95 % der Theorie). Schmelzpunkt 248° C (unter Zersetzung).

Beispiel 8:

2-Fluor-1,1-dimethyl-ethylamin

Zu 63,75 g (0,5 Mol) 2-Fluor-1,1-(dimethyl)-ethylamin-Hydrochlorid aus Beispiel 7 werden 60 g 45 %ige wäßrige NaOH bei einer Ölbadtemperatur von 100° C getropft. Gleichzeitig wird das freigesetzte Amin abdestilliert. Nach Trocknen über Magnesiumsulfat wird redestilliert. Man erhält an Produkt 2-Fluor-1,1-dimethyl-ethylamin. Ausbeute 41,4 g (91 % der Theorie). Siedepunkt: 82 - 84° C (bei Normaldruck), $n_D^{20}$ : 1,4042.

Beispiel 9:

2-Fluor-1-(monofluormethyl)-1-methyl-ethylamin-Hydrochlorid

Analog Beispiel 7 erhält man aus 67,5 g (0,5 Mol) 2-Fluor-1-(monofluormethyl)-1-methylisocyanat, das nach Beispiel 5 hergestellt wird, 62,5 g (86 % der Theorie) 2-Fluor-1-(monofluormethyl)-1-methyl-ethylamin-Hydrochlorid Schmelzpunkt 214° C (unter Zersetzung).

Beispiel 10:

2-Fluor-1-(monofluormethyl)-1-methyl-ethylamin

Analog Beispiel 8 erhält man aus 72,25 g (0,5 Mol) 2-Fluor-1-(monofluormethyl)-1-methyl-ethylamin-Hydrochlorid aus Beispiel 9 50,7 g (93 % der Theorie) 2-Fluor-1-(monofluormethyl)-1-ethylamin vom Siedepunkt 91 - 92° C (bei Normaldruck), $n_D^{20}$ : 1,3892.

Beispiel 11:

2-Fluor-1,1-bis-(monofluormethyl)-ethylamin-Hydrochlorid

Analog Beispiel 7 erhält man aus 76,5 g (0,5 Mol) 2-Fluor-1,1-bis-(monofluormethyl)-isocyanat, das nach Beispiel 6 hergestellt wird, 75 g (92 % der Theorie) 2-Fluor-1,1-bis-(monofluormethyl)-ethylamin-Hydrochlorid. Schmelzpunkt 220 ° C (unter Zersetzung).

Beispiel 12:

2-Fluor-1,1-bis-(monofluormethyl)-ethylamin

Analog Beispiel 8 erhält mana us 81,75 g (0,5 Mol) 2-Fluor-1,1-bis-(monofluormethyl)-ethylamin-Hydrochlorid aus Beispiel 11 54,6 g (86 % der Theorie) 2-Fluor-1,1-bis-(monofluormethyl)-ethylamin vom Siedepunkt 120 - 122 ° C (bei Normaldruck) und $n_D^{20}$ : 1,3813.

## Patentansprüche

1. Fluorierte tertiäre Butylamine der allgemeinen Formel (I)

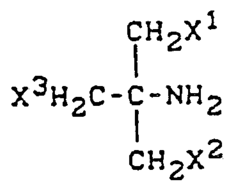

(I)

in welcher die Reste $X^1$ bis $X^3$ gleich oder verschieden sein können und für Wasserstoff oder Fluor stehen,
wobei wenigstens einer der Reste $X^1$ bis $X^3$ für Fluor stehen muß.

2. 2-Fluor-1,1-dimethyl-ethylamin.

3. 2-Fluor-1-(monofluormethyl)-1-methyl-ethylamin.

4. 2-Fluor-1,1-bis-(monofluormethyl)-ethylamin.

5. Verfahren zur Herstellung von fluorierten tertiären Butylaminen der allgemeinen Formel (I)

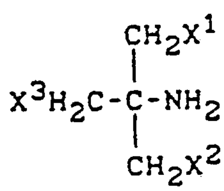

(I)

in welcher die Reste $X^1$ bis $X^3$ gleich oder verschieden sein können und für Wasserstoff oder Fluor stehen,
wobei wenigstens einer der Reste $X^1$ bis $X^3$ für Fluor steht,

dadurch gekennzeichnet, daß man in der Herstellungsvariante A)

(a) die halogenierten Pivalsäurehalogenide der allgemeinen Formel (II)

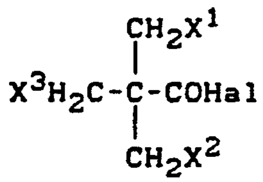

(II),

in welcher $X^1$ bis $X^3$ die oben angegebene Bedeutung haben und Hal für Fluor oder Chlor steht,

mit einem Azidgruppen übertragenden Reagenz in die fluorierten Pivalsäureazide der allgemeinen Formel (III)

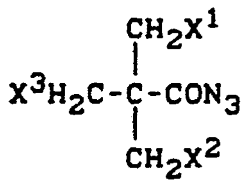

(III),

in welcher $X^1$ bis $X^3$ die oben angegebene Bedeutung haben, umsetzt und
(b) die gegebenenfalls isolierten Verbindungen der allgemeinen Formel (III) thermisch zu den fluorierten Isocyanaten der allgemeinen Formel (IV)

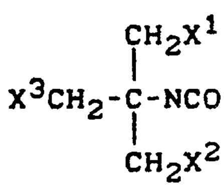

(IV),

in welcher $X^1$ bis $X^3$ die oben angegebene Bedeutung haben, zersetzt und
(c) die gegebenenfalls isolierten Verbindungen der allgemeinen Formel (IV) durch saure Hydrolyse in die fluorierten tertiären Butylamin-Säureadditionssalze der allgemeinen Formel (V)

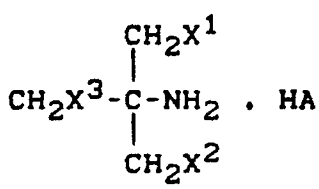

(V),

in welcher $X^1$ bis $X^3$ die oben angegebene Bedeutung haben, und A für den einwertigen Rest einer Säure steht, umwandelt und
(d) die gegebenenfalls isolierten Verbindungen der allgemeinen Formel (V) durch Behandlung mit Base in die fluorierten tertiären Butylamine der allgemeinen Formel (I) überführt,

oder

daß man in der Verfahrensvariante B)

die Isocyanate der Formel (IV)

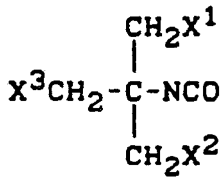

(IV),

in welcher $X^1$ bis $X^3$ die oben angegebene Bedeutung haben,

durch alkalische Hydrolyse direkt in die fluorierten tertiären Butylamine der allgemeinen Formel (I) überführt,

oder daß man in der Verfahrensvariante C)

Pivalsäureamide der allgemeinen Formel (VI)

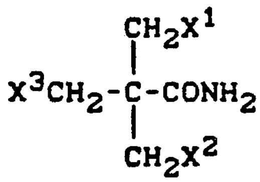

(VI),

in welcher $X^1$ bis $X^3$ die oben angegebene Bedeutung haben,

mit Hypohalogenit umsetzt.

## Claims

1. Fluorinated tertiary butylamines of the general formula (I)

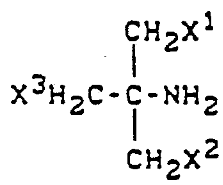

(I)

in which the radicals $X^1$ to $X^3$ can be identical or different and represent hydrogen or fluorine, but at least one of the radicals $X^1$ to $X^3$ must represent fluorine.

2. 2-Fluoro-1,1-dimethyl-ethylamine.

3. 2-Fluoro-1-(monofluoromethyl)-1-methylethylamine.

4. 2-Fluoro-1,1-bis-(monofluoromethyl)-ethylamine.

5. Process for the preparation of fluorinated tertiary butylamines of the general formula (I)

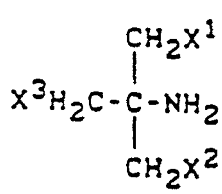

(I)

in which the radicals $X^1$ to $X^3$ can be identical or different and represent hydrogen or fluorine, and at least one of the radicals $X^1$ to $X^3$ represents fluorine,

characterised in that, in preparation variant A),

(a) the halogenated pivalic acid halides of the general formula (II)

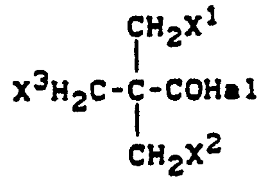

(II)

in which $X^1$ to $X^3$ have the abovementioned meaning and Hal represents fluorine or chlorine,

are reacted with a reagent which donates azido groups into the fluorinated pivalic acid azides of the general formula (III)

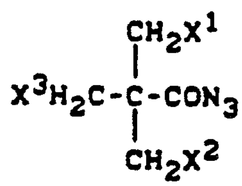

(III)

in which $X^1$ to $X^3$ have the abovementioned meaning,
and
(b) the optionally isolated compounds of the general formula (III) are decomposed by means of heat to give the fluorinated isocyanates of the general formula (IV)

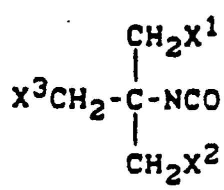

(IV)

in which $X^1$ to $X^3$ have the abovementioned meaning,
and
(c) the optionally isolated compounds of the general formula (IV) are converted by acid hydrolysis into the fluorinated tertiary butylamine acid addition salts of the general formula (V)

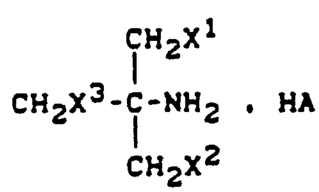

(V)

in which $X^1$ to $X^3$ have the abovementioned meaning and A represents the monovalent radical of an acid, and
(d) the optionally isolated compounds of the general formula (V) are converted by treatment with a

base into the fluorinated tertiary butylamines of the general formula (I),

or in that, in process variant B),

the isocyanates of the formula (IV)

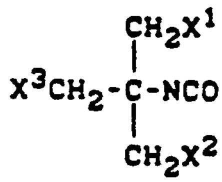

(IV)

in which $X^1$ to $X^3$ have the abovementioned meaning,

are converted directly by alkaline hydrolysis into the fluorinated tertiary butylamines of the general formula (I),

or in that, in process variant C),

pivalic acid amides of the general formula (VI)

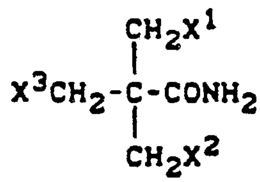

(VI)

in which $X^1$ to $X^3$ have the abovementioned meaning,

are reacted with hypohalite.

## Revendications

1. Tertio-butylamines fluorées de formule générale (I)

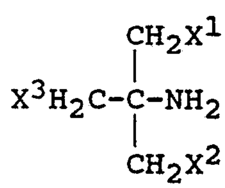

(I)

dans laquelle les restes $X^1$ à $X^3$ peuvent être identiques ou différents et représentent de l'hydrogène ou du fluor,
l'un au moins des restes $X^1$ à $X^3$ représentant obligatoirement du fluor.

2. La 2-fluoro-1,1-diméthyléthylamine.

3. La 2-fluoro-1-(monofluorométhyl)-1-méthyl-éthylamine.

4. La 2-fluoro-1,1-bis-(monofluorométhyl)-éthylamine.

5. Procédé de production de tertio-butylamines fluorées de formule générale (I)

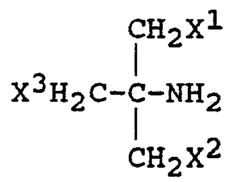

(I)

dans laquelle les restes $X^1$ à $X^3$ peuvent être identiques ou différents et représentent de l'hydrogène ou du fluor,

l'un au moins des restes $X^1$ à $X^3$ représentant du fluor,

caractérisé en ce que, dans la variante de préparation A)

(a) les halogénures d'acides pivaliques halogénés de formule (II)

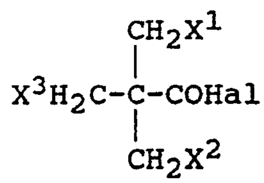

(II)

dans laquelle $X^1$ à $X^3$ ont la définition indiquée ci-dessus et Hal représente du fluor ou du chlore, sont amenés à réagir avec un réactif de transfert de groupes azide pour former les azides d'acides pivaliques fluorés de formule générale (III)

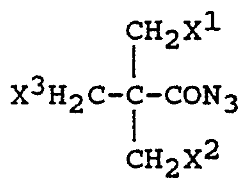

(III)

dans laquelle $X^1$ à $X^3$ ont la définition indiquée ci-dessus et

(b) les composés éventuellement isolés de formule générale (III) sont décomposés thermiquement en les isocyanates fluorés de formule générale (IV)

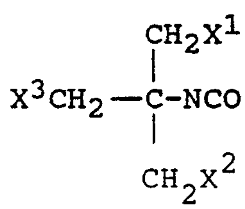

(IV)

dans laquelle $X^1$ à $X^3$ ont la définition indiquée ci-dessus et

(c) les composés éventuellement isolés de formule générale (IV) sont convertis par hydrolyse acide en les sels d'addition d'acide de tertio-butylamines fluorées de formule générale (V)

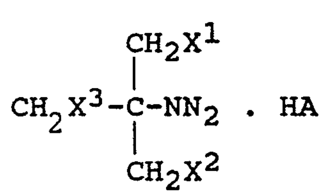

(V)

dans laquelle $X^1$ à $X^3$ ont la définition indiquée ci-dessus et A représente le reste monovalent

d'acide et

(d) les composés éventuellement isolés de formule générale (V) sont transformés par traitement avec une base en les tertio-butylamines fluorées de formule générale (I),

ou bien en ce que

dans la variante B) du procédé

les isocyanates de formule (IV)

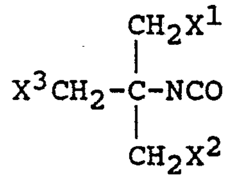

(IV)

dans laquelle $X^1$ à $X^3$ ont la définition indiquée ci-dessus

sont convertis directement par hydrolyse alcaline en les tertio-butylamines fluorées de formule générale (I)

ou en ce que dans la variante C) du procédé on fait réagir avec un hypohalogénite des amides d'acides pivaliques de formule générale (VI)

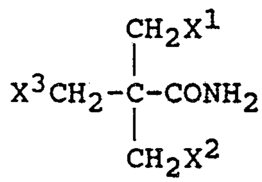

(VI)

dans laquelle $X^1$ à $X^3$ ont la définition indiquée ci-dessus.